# EUROPEAN PATENT SPECIFICATION

(11) **EP 0 614 741 B1**
(45) Date of publication and mention of the grant of the patent: **03.12.1997**
(21) Application number: 94103231.0
(22) Date of filing: 03.03.1994
(51) Int. Cl.: B29C 45/00, A61F 13/26

(54) **Method of making an elongate component and injection mold assembly for making the same**
Verfahren zur Herstellung eines länglichen Gegenstandes und Spritzgiessvorrichtung zu dessen Herstellung
Procédé de fabrication d'un article allongé et dispositif de moulage par injection

(30) Priority: 04.03.1993 US 27311
(43) Date of publication of application: 14.09.1994
(73) Proprietor: McNEIL-PPC, INC., Milltown New Jersey 08850 (US)
(72) Inventor: Babinski, Carl P., Stockton, NJ 08559 (US)
(74) Representative: Strehl Schübel-Hopf Groening & Partner

(56) References cited:
- EP-A- 0 291 024
- EP-A- 0 438 672
- FR-A- 1 305 166
- GB-A- 1 038 897
- US-A- 3 830 236
- US-A- 3 954 104
- US-A- 4 361 150
- US-A- 4 743 420
- US-A- 5 002 526

## Description

### BACKGROUND OF THE INVENTION

### Field of the Invention

This invention relates to a method of making an elongated component for a catamenial tampon applicator in accordance with claim 1 and to an injection mold assembly for making an elongated component in accordance with claim 6. Such a thin walled plastic product is designed to be quickly biodegradable.

### Description of the Prior Art

A method and an injection mold assembly according to the preamble of claims 1 and 6 is known from US-A-4 361 150. This document discloses a method of making an elongated component for a tampon applicator, wherein said component has a thin cylindrical wall which is provided with an annular ring at one end and with petals at the forward dome-shaped end of the component comprising: providing a mold assembly having an elongated mold insert which is insertable between first and second mold halves so as to define an elongated mold cavity that corresponds to the shape of the applicator component; said injection mold assembly having a melt flow channel defined therein; the melt flow channel extends in the enlongated direction; the thickness of said melt flow channel is greater than the thickness of the adjacent portion of the mold cavity; injecting a solidifiable liquid material into the elongated mold cavity and removing the formed component from the injection mold once the material has solidified.

FR-A-1 305 166 discloses a method of making tubes by injection molding in only one step to reduce at the same time the wall thickness and to realize an economical use of the material. As to solve this problem, this document proposes longitudinal bridges within the outer surface within the elongated mold insert to provide longitudinal grooves in the outer surface of the tube. During injection, the material flows to the basis of the tube and forms longitudinal ribs at the outer surface of the tube. These grooves are used as a multiplicity of injection channels.

EP-A-0 291 024 is related to tampon applicators and compositions for making same. This document discloses a biodegradable tampon applicator comprising a molded, flexible polymeric hollow cylindrical body for enclosing a tampon therein and having a first open end through which the tampon is expelled and a second open end for receiving a plunger to expell said tampon, wherein said body comprising a moldable poly 3-hydroxybutyric acid composition.

US-A-3 954 104 discloses a thermoplastic water-dispersible, biodegradable composition which is resistant to fungal attack prior to dispersal and containers made therefrom. The composition comprises hydroxy-alkyl cellulose, starch, and a member selected from the group consisting of sorbic acid and its alkali metal salts as the anti-fungal agent. The hydroxy-alkyl cellulose is a biodegradable, thermoplastic polymer selected to be water-soluble and being present in a proportion sufficient to make the composition water-dispersible and thermoplastic. Starch is a biodegradable filler to achieve rigidity, little affect of high humidity and low costs of the product.

US-A-5 002 526 is related to a tampon applicator being biodegradable and water-dispersible. The applicator is formed by injection molding a modified poly(vinylalcohol) which is self-plasticizing.

In response to growing concern over the environment, manufacturers of plastic products such as tampon applicators are endeavoring to fabricate those components out of materials and in configurations which will enhance biodegradability.

The biodegradability of a plastic component will be enhanced by making the walls of the component as thin as possible, because environmental forces will break down a thin object much quicker than a thicker object. Biodegradability can also be enhanced by using specialized materials, such as starch based or starch filled plastics, polyvinyl alcohol or PHBV. Unfortunately, it has been difficult to simultaneously reduce product wall thickness and use such biodegradable polymer materials, because such materials as a group have a lower melt index than the less environmentally-friendly polymers they are intended to replace. A material with a low melt index has a high viscosity when heated to a liquid state, making it difficult to fill a mold quickly and without degradation.

It is clear that there has existed a long and unfilled need in the prior art for an improved method for fabricating a biodegradable plastic component, such as an applicator for a tampon assembly, out of a degradable polymer which permits the component to have thinner walls than heretofore possible, thereby achieving an even higher level of biodegradability.

### SUMMARY OF THE INVENTION

Accordingly, it is an object of the invention to provide a method for molding a biodegradable plastic product such as an applicator for a tampon assembly which makes it possible to mold thin product parts out of a biodegradable polymer which has a relatively low melt index.

It is further an object of the invention to provide an improved thin walled plastic component such as an applicator for a tampon assembly which is fabricated out of a biodegradable polymeric material.

The above objects are achieved according to the invention by a method as specified in claim 1. Accordingly the method of making an elongated component for a tampon applicator includes steps of (a) providing an injection mold which defines an elongated mold cavity that corresponds to the desired shape of the component, the injection mold having a melt flow channel defined therein which extends in the elongated direction, said melt flow channel having a thickness which is greater than the thickness of the adjacent portion of the mold cavity; (b) injecting a solidifiable liquid material into the elongated mold cavity; and (c) removing the formed component from the injection mold once the material has solidified, the melt flow channel enabling the component to be formed with thinner walls, and with more viscous liquid material than was heretofore possible.

Furthermore, the invention is related to an injection mold assembly in accordance with the preamble of claim 6 disclosing features of the above-mentioned document US-A-4 361 150. Accordingly, this document discloses an injection mold assembly for making an elongated component for a tampon applicator, wherein said component has a thin cylindrical wall which is provided with an annular ring at one end and with petals at the forward dome-shaped end of the component and wherein said mold assembly having an enlongated mold insert which is insertable between first and second mold halves so as to define an enlogated mold cavity which corresponds to the shape of the applicator component. In accordance with the present invention, the above objects are achieved by an injection mold assembly as disclosed in claim 4. According to this second aspect of the invention, said injection mold assembly for making an elongated component for a tampon applicator includes an injection mold which defines an elongated mold component, the injection mold having a melt flow channel defined therein which extends in the elongated direction, the melt flow channel having a thickness which is greater than the thickness of the adjacent portion of the mold cavity; and structure for injecting a solidifiable liquid material into the elongated mold cavity, whereby the melt flow channel enables the component to be formed with thinner walls and with more viscous liquid material than was heretofore possible.

Particular embodiments of the invention are disclosed in the dependent claims.

For a better understanding of the invention, its advantages, and the objects obtained by its use, reference should be made to the drawings which form a further part hereof, and to the accompanying descriptive matter, in which there is illustrated and described a preferred embodiment of the invention.

### BRIEF DESCRIPTION OF THE DRAWINGS

FIGURE 1 is a diagrammatical cross sectional view through a mold assembly according to a preferred embodiment of the invention;
FIGURE 2 is a longitudinal cross sectional view through an improved tampon applicator component according to the preferred embodiment of the invention; and
FIGURE 3 is a transverse cross sectional view through the tampon applicator component depicted in FIGURE 2.

### DETAILED DESCRIPTION OF THE PREFERRED EMBODIMENT(S)

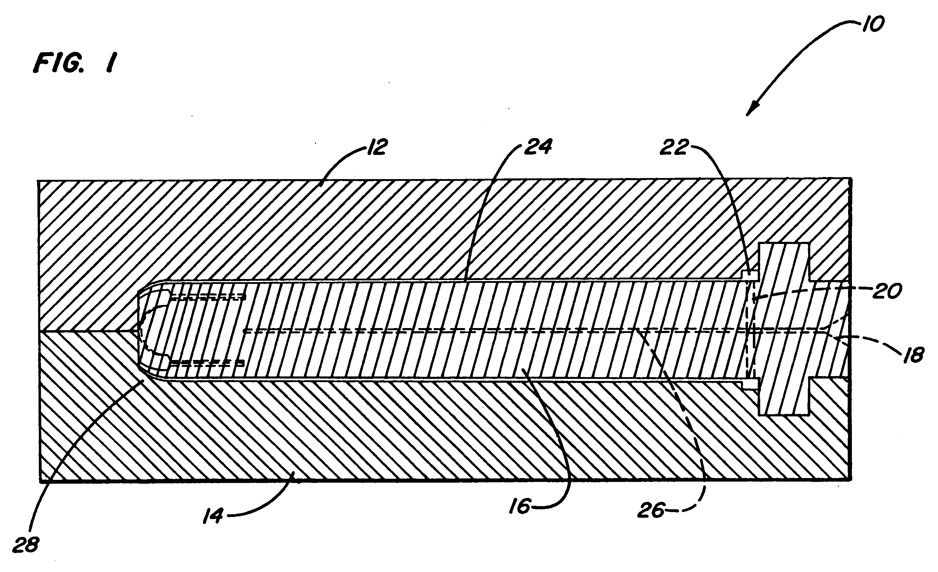

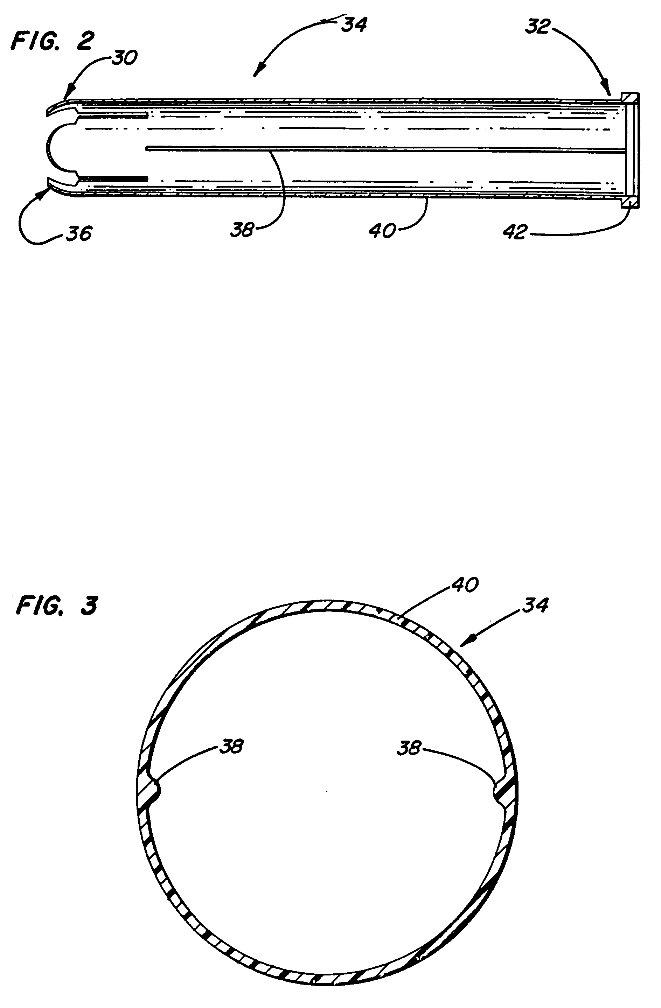

Referring now to the drawings, wherein like reference numerals designate corresponding structure throughout the views, and referring in particular to FIGURES 1 and 2, an injection mold assembly 10 for making an elongated component 34 for a tampon applicator includes a first mold half 12, a second mold half 14 and an elongate mold insert 16 which is insertable between first and second mold halves 12, 14 so as to define an elongated mold cavity which corresponds to the desired shape of the applicator component 34. As may be seen in FIGURE 1, mold insert 16 includes a passage 20 and a gate 18 defined therein for introducing molten thermoplastic material to the mold cavity during the injection molding process. An exit gate (not shown) is also provided at an opposite end of the mold cavity for permitting displaced air to escape from the mold cavity during the molding process. It is to be understood that the mold assembly 10 depicted in FIGURE 1 is exemplary only, and that a mold assembly according to the invention could take other embodiments as well.

Looking again to FIGURES 1-3, the elongated mold cavity includes an annular space 22 which is used to form an annular end ring 42 on the tampon applicator component 34. The mold cavity also includes a thin cylindrical space 24 which extends for substantially the entire length of the mold cavity and terminates at a front end portion 28, as may be seen in FIGURE 1. Thin cylindrical space 24 is used to mold the thin cylindrical wall 40 of applicator component 34, while front end portion 28 is shaped to mold the forward end 30 of component 34, including the petals 36 which are typically formed on such an applicator component 34.

According to one important aspect of the invention, a melt flow channel 26 is defined in mold insert 16 so as to be part of the elongated mold cavity. Melt flow channel 26 has a thickness which is greater than the thickness of the adjacent thin cylindrical space 24 in the mold cavity. Melt flow channel 26 extends in the longitudinal direction of the mold cavity, and preferably is substantially parallel to the longitudinal axis of mold insert 16, and, thus, the longitudinal axis of the applicator component 34 being formed. More than one such melt flow channel 26 may be defined in mold assembly 10, and, preferably, gate 18 is in direct communication with melt flow channel 26.

In operation, a heated liquid thermoplastic polymer is introduced into the mold cavity through gate 18 and passage 20. Preferably, the polymer is a biodegradable polymer, such as a starch based polymer, a starch filled polymer, polyvinyl alcohol or PHBV, which stands for polyhydroxybutyratevalerate copolymer. Most preferably, the polymer material is PHBV. These biodegradable polymers, as a group, have a relatively low melt index, and are therefore more viscous and harder to injection mold, particularly in mold cavities which, include long thin spaces such as the thin cylindrical space 24 in the mold cavity depicted in FIGURE 1. During the molding process, the melt flow channels 26 help carry the viscous, hot, thermoplastic along the length of the mold cavity, as the material simultaneously fans out to fill the thin cylindrical space 24. Through use of the melt flow channels 26, a longer, wider, or larger diameter part may be molded at a given wall thickness than was previously attainable. The invention can be practiced to produce products having length to thickness ratios from 1-100 to one. The specific length to thickness ratio will depend on the melt index of the plastic being injected; the 100 to one length to thickness ratio should be possible at a melt index of 35. As a result, the total biodegradability characteristics of the product are enhanced beyond what was previously achievable.

In addition, the invention could be used to mold components out of conventional thermoplastic materials which are longer, wider or larger at given wall thicknesses than were previously possible. For example, the invention could be used to mold synthetic polymers such as polyethylene, UHMWPE, HDPE, LDPE, LLDPE, elastomers, PVC, PP, PET, nylon, polystyrene, polycarbonate, thermoplastic polyurethane and thermoset polyesters.

### 1. Example 1

Three elongated plastic components having the same shape and wall thickness were fabricated by injection molding using a standard mold having a gate at one end, a standard mold having a gate positioned in the center of the mold, and with a mold according to the invention having two melt flow channels formed therein. Low density polyethylene was used for each of the fabricated components. To form a viable component using the standard injection mold having a gate at one end, it was found that an injection pressure of 11031.47·10³Pa (1600 psi) was necessary at a temperature of 232.22°C (450°F). To fabricate a viable component using the central gate pusher, it was found that an injection pressure of 6849.67·10³Pa (1000 psi) was necessary at a melt temperature of 232.22°C (450°F). When the same component was fabricated using a mold according to the invention with a gate at the end of the mold, the necessary injection pressure and temperature were both reduced. It was found that a viable component could be fabricated at an injection pressure of 5515.74·10³Pa (800 psi) with a melt temperature of 162.78°C (325°F). This was a clear improvement not only over the standard end gated mold, but over the center gated mold as well.

### 2. Example 2

Three elongated biodegradeable plastic components having the same wall thickness and shape were fabricated out of polyhydroxlbutylvalerate ("PHBV"). As in the first example, the components were fabricated and, respectively, a standard mold having a gate at one end, a standard mold having a gate at the center of the mold, and with a mold according to the invention having a gate at one end. Using the standard mold having a gate at one end, a somewhat less than viable component was produced at a pressure of 10342.00·10³Pa (1500 psi) at a melt temperature of 146.11°C (295°F). A more viable component was produced using the standard mold having a gate at the center at an injection pressure of 7280.77·10³Pa (1056 psi), at melt temperature of 135°C (275°F). Using a mold according to the invention, a viable component was produced at an injection pressure of 6.549.94·10³Pa (950 psi) at a melt temperature 143.33°C (290°F).

**TABLE 1**

| **MELT FLOW CHANNEL DATA** | | | |
|---|---|---|---|
| | Std. End Gate Pusher | Std. Cen. Gate Pusher | M.F. Chan Pusher |
| | | | |

| **LDPE** **Example 1** | | | |
|---|---|---|---|
| Injection Pressure | (1600 psi) 11.037.47·10³Pa | (1000 psi) 6849.67·10³Pa | (800 psi) 5515.74·10³ Pa |
| Melt Temp. | 232.33°C (450°F) | 232.33°C (450°F) | 162.78°C (325°F) |
| Melt Flow Index | 35 | 35 | 35 |

| **PHBV** **Example 2** | | | |
|---|---|---|---|
| Injection Pressure | (1500 psi) 10342.00·10³Pa | (1056 psi) 7280.77·10³Pa | (950 psi) 6549.94·10³Pa |
| Melt Temp. | 146.11°C (295°F) | 135.00°C (275°F) | 143.33°C (290°F) |
| Melt Flow Index | <1 | <1 | <1 |

It is to be understood, however, that even though numerous characteristics and advantages of the present invention have been set forth in the foregoing description, together with details of the structure and function of the invention, the disclosure is illustrative only, and changes may be made in detail, especially in matters of shape, size and arrangement of parts within the principles of the invention to the full extent indicated by the broad general meaning of the terms in which the appended claims are expressed.

## Claims

1. Method of making an elongate component (34) for a catamenial tampon applicator consisting of said elongate component (34) and a pusher element for the tampon, wherein
- said component (34) has a thin cylindrical wall (40) which is provided with
- an annular ring (42) at one end and with
- petals (36) at the forward dome-shaped end (28) of the component (34) comprising:
- a mold assembly (10) having an elongate mold insert (16) being insertable between first and second mold halves (12, 14) so as to define an elongate annular mold cavity corresponding to the shape of the applicator component (34);
- said injection mold assembly (10) having a melt flow channel defined therein; and
- the melt flow channel extending in the longitudinal direction of the mold assembly;
- injecting a solidifiable liquid thermoplastic polymer into the elongate mold cavity; and
- removing the formed component (34) from the injection mold once the material has solidified,
characterized by
- a heated liquid therm plastic biodegradable polymer having a melt index of equal or less than 35 being introduced through a gate (18) and a passage (20) of said mold insert (16) into at least one melt flow channel (26) provided in the outer surface of the insert (16) and into
- a narrow cylindrical annular space (24) in the mold cavity, the thickness of said melt flow channels (26) being greater than the thickness of the adjacent portion of the mold cavity, helping to carry the viscous, hot, thermoplastic polymer along the length of the mold cavity, as the material simultaneously fans out to fill the narrow cylindrical annular space (24) to produce said applicator component (34).

2. Method according to claim 1, characterized by injecting low density polyethylene polymer at an injection pressure of 5515.74 . 10³ Pa (800 psi) with a melt temperature of 162.78°C (325°F).

3. Method according to claim 1, characterized by injecting a polyhydroxylbutylvalerate copolymer at an injection pressure of 6549.94 . 10³ Pa (950 psi), at a melt temperature of 143.33°C (290°F).

4. Injection mold assembly (10) for making an elongate component (34) for a tampon applicator consisting of said elongate component (34) and a pusher element for the tampon from a thermoplastic polymer, wherein said component (34) has a thin cylindrical wall (40) which is provided with an annular ring (42) at one end and with petals (36) at the forward dome-shaped end (28) of the component (34) and wherein
- said mold assembly (10) having
- an elongate mold insert (16) which is insertable between first and second mold halves (12, 14) so as to define an
- elongate mold cavity having a cylindrical annular space (24) which corresponds to the shape of the applicator component (34), and a melt flow channel extending in the longitudinal direction of the mold assembly, characterised by
- at least one melt flow channel (26) being defined in the outer surface of said mold insert (16) and extending in the longitudinal direction of the mold cavity, and the mold insert (16) including a gate (18) and a passage (20) defined therein, through which the polymer being injectable into the narrow cylindrical annular space (24) of the mold cavity, wherein said at least one melt flow channel (26) having a thickness which is greater than the thickness of the adjacent cylindrical annular space (24) in the mold cavity.

## Patentansprüche

1. Verfahren zur Herstellung einer sich längserstreckenden Komponente (34) für einen katamenialen Tamponapplikator, der aus der sich längserstreckenden Komponente (34) und einem Ausstoßelement für den Tampon besteht, wobei
- die Komponente (34) eine dünne, zylindrische Wand (40) hat, die mit
- einem Ring (42) an einem Ende und mit
- Zungen (36) an dem vorderen gewölbten Ende (28) der Komponente (34) versehen ist, umfassend:
- eine Spritzformanordnung (10) mit einem sich längserstreckenden Spritzformeinsatz (16), der zwischen ersten und zweiten Formhälften (12, 14) einsetzbar ist, um einen sich längserstreckenden, ringförmigen Formhohlraum zu bilden, der der Form der Applikatorkomponente (34) entspricht;
- die Spritzformanordnung (10) hat einen Schmelzflußkanal, der darin gebildet ist; und
- der Schmelzflußkanal erstreckt sich in Längsrichtung der Spritzformanordnung erstreckt;
- einerstarrungsfähiges, flüssiges, thermoplastisches Polymer in den sich längserstrekkenden Formhohlraum eingespritzt wird; und
- die geformte Komponente (34) aus der Spritzform entfernt wird, wenn der Werkstoff erstarrt ist,
dadurch gekennzeichnet, daß
- ein erhitztes, flüssiges, thermoplastisches, biologisch abbaubares Polymer mit einem Schmelzindex von gleich oder weniger als 35 durch einen Eingußkanal (18) und einen Verbindungskanal (20) des Spritzformeinsatzes (16) in mindestens einen, in der äußeren Oberfläche des Spritzformeinsatzes (16) vorgesehenen Schmelzflußkanal (26) und in
- einen engen zylindrischen, ringförmigen Zwischenraum (24) in dem Formhohlraum eingeleitet wird, wobei die Form des Schmelzflußkanals (26) größer als die Form des benachbarten Teils des Formhohlraums ist und das Fließen des viskosen, heißen, thermoplastischen Polymers über die Länge des Formhohlraums unterstützt, wenn sich das Material gleichzeitig ausbreitet, um den engen, zylindrischen, ringförmigen Zwischenraum (24) zur Herstellung der Applikatorkomponente (34) zu füllen.

2. Verfahren nach Anspruch 1, gekennzeichnet durch das Einspritzen eines Polyethylen-Polymerisats geringer Dichte bei einem Einspritzdruck von 5515,74 . 10³ Pa (800 psi) bei einer Schmelztemperatur von 162,78°C (325°F).

3. Verfahren nach Anspruch 1, gekennzeichnet durch das Einspritzen eines Polyhydroxylbutylvalerat-Copolymerisats bei einem Einspritzdruck von 6549,94 . 10³ Pa (950 psi) und einer Schmelztemperatur von 143,33°C (290°F).

4. Spritzformanordnung (10) zur Herstellung einer sich längserstreckenden Komponente (34) für einen aus der sich längserstreckenden Komponente (34) und einem Ausstoßelement für den Tampon bestehenden Tamponapplikator, aus einem thermoplastischen Polymer, wobei die Komponente (34) eine dünne, zylindrische Wand (40) hat, die mit einem Ring (42) am einen Ende und mit Zungen (36) an dem vorderen, gewölbten Ende (28) der Komponente (34) versehen ist, und
- die Spritzformanordnung (10)
- einen sich längserstreckenden Spritzformeinsatz (16) hat, der zwischen ersten und zweiten Formhälften (12, 14) einsetzbar ist, um
- einen sich längserstreckenden Formhohlraum mit einem zylindrischen, ringförmigen Zwischenraum, der der Form der Applikatorkomponente (34) entspricht, sowie einen Schmelzflußkanal zu bilden, der sich in Längsrichtung der Spritzformanordnung erstreckt,
gekennzeichnet durch
- mindestens einen Schmelzflußkanal (26), der in der äußeren Oberfläche des Spritzformeinsatzes (16) gebildet ist und sich in Längsrichtung des Formhohlraums erstreckt, und den Spritzformeinsatz (16) mit einem Eingußkanal (18) und einem darin vorhandenen Verbindungskanal (20), durch die das Polymer in den engen, zylindrischen, ringförmigen Zwischenraum (24) des Formhohlraums einspritzbar ist, wobei der mindestens eine Schmelzflußkanal (26) eine Form hat, die größer als die Form des benachbarten, zylindrischen, ringförmigen Zwischenraums (24) in dem Formhohlraum ist.

## Revendications

1. Procédé de fabrication d'un élément allongé (34) pour applicateur de tampon cataménial comportant ledit élément allongé (34) et un organe formant dispositif de poussée du tampon, dans lequel
- ledit article (34) a une fine paroi cylindrique (40) qui est munie :
- d'un anneau annulaire (42) au niveau d'une première extrémité et de
- pétales (36) au niveau de l'extrémité avant (28) en forme de dôme de l'élément (34) comportant :
- un ensemble de moule (10) ayant un élément rapporté de moule allongé (16) pouvant être inséré entre des première et seconde moitiés de moule (12, 14) de manière à définir une cavité de moule annulaire allongée correspondant à la forme de l'élément pour applicateur (34),
- ledit ensemble de moule d'intection (10) ayant un canal d'écoulement de matière fondue défini à l'intérieur de celui-ci ; et
- le canal d'écoulement de matière fondue s'étend dans la direction longitudinale de l'ensemble de moule ;
- on injecte un polymère thermoplastique liquide pouvant être solidifié à l'intérieur de la cavité de moule allongée ; et
- on enlève l'élément formé (34) à partir du moule d'injection, une fois que le matériau a été solidifié,
caractérisé en ce
- un polymère biodégradable thermoplastique liquide chauffé, ayant un indice de fusion inférieur ou égal à 35, est introduit à travers une porte (18) et un passage (20) dudit élément rapporté de moule (16) jusqu'à au moins un canal d'écoulement de matière fondue (26) agencé dans la surface extérieure de l'élément rapporté (16), et dans
- un espace annulaire cylindrique étroit (24) de la cavité de moule, l'épaisseur desdits canaux d'écoulement de matière fondue (26) étant plus grande que l'épaisseur de la partie adjacente de la cavité de moule, aidant à supporter les polymères thermoplastiques chauds, visqueux sur toute la longueur de la cavité de moule, lorsque le matériau s'écoule de manière simultanée pour remplir l'espace annulaire cylindrique étroit (24) pour fournir ledit élément pour applicateur (34).

2. Procédé selon la revendication 1, caractérisé en ce qu'il comporte l'étape consistant à intecter du polymère de polyéthylène faible densité à une pression d'intection de 5515,74 · 10 Pa (800 psi) à une température de fusion de 162,78°C (325°F).

3. Procédé selon la revendication 1, caractérisé en ce qu'il comporte l'étape consistant à injecter un copolymère de polyhydroxylbutylvalérate à une pression d'intection de 6549,94 · 10 Pa (950 psi) à une température de fusion de 143,33°C (290°F).

4. Ensemble de moule d'injection (10) pour réaliser un élément allongé (34) pour applicateur de tampon constitué dudit élément allongé (34) et d'un organe formant dispositif de poussée du tampon, à partir d'un polymère thermoplastique, dans lequel ledit élément (34) a une paroi cylindrique fine (40) qui est munie d'une boucle annulaire (42) au niveau d'une première extrémité et de pétales (36) au niveau de l'extrémité avant (28) en forme de dôme de l'élément (34) et dans lequel :
- ledit ensemble de moule (10) comporte
- un élément rapporté de moule allongé (16) qui peut être inséré entre des première et seconde moitiés de moule (12, 14) de manière à définir
- une cavité de moule allongée ayant un espace annulaire cylindrique (24) qui correspond à la forme de l'élément pour applicateur (34), et un canal d'écoulement de matière fondue s'étendant dans la direction longitudinale de l'ensemble de moule, caractérisé en ce que
- au moins un canal d'écoulement de matière fondue (26) est défini dans la surface extérieure dudit élément rapporté de moule (16) et s'étend dans la direction longitudinale de la cavité de moule, et en ce que l'élément rapporté de moule (16) comporte une porte (18) et un passage (20) définis dans celuici, à travers lesquels le polymère peut être injecté dans l'espace annulaire cylindrique étroit (24) de la cavité de moule, dans lequel ledit au moins un canal d'écoulement de matière fondue (26) a une épaisseur qui est plus grande que l'épaisseur de l'espace annulaire cylindrique adjacent (24) de la cavité de moule.
